(19) Europäisches Patentamt
European Patent Office
Office européen des brevets

(11) EP 3 110 406 B1

# (12) EUROPEAN PATENT SPECIFICATION

(45) Date of publication and mention of the grant of the patent:
**04.04.2018 Bulletin 2018/14**

(21) Application number: **15705830.6**

(22) Date of filing: **24.02.2015**

(51) Int Cl.:
***A61K 9/00*** *(2006.01)* ***A61K 31/519*** *(2006.01)*
***A61K 9/20*** *(2006.01)*

(86) International application number:
**PCT/EP2015/053764**

(87) International publication number:
**WO 2015/128298 (03.09.2015 Gazette 2015/35)**

## (54) PHARMACEUTICAL COMPOSITION

PHARMAZEUTISCHE ZUSAMMENSETZUNG ENTHALTEND TRAMETINIB

COMPOSITION PHARMACEUTIQUE COMPRENANT DE LA TRAMETINIB

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2014 EP 14157041**

(43) Date of publication of application:
**04.01.2017 Bulletin 2017/01**

(73) Proprietor: **ratiopharm GmbH**
**89079 Ulm (DE)**

(72) Inventors:
- **LEUTNER, Dirk**
  **67000 Strasbourg (FR)**
- **MIKA, Hans Juergen**
  **53229 Bonn (DE)**
- **STROHMEYER, Jutta**
  **85356 Freising (DE)**

(74) Representative: **Teipel, Stephan et al**
**Lederer & Keller**
**Patentanwälte Partnerschaft mbB**
**Unsöldstrasse 2**
**80538 München (DE)**

(56) References cited:
**WO-A1-2012/027438 WO-A2-2012/088033**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 110 406 B1

## Description

The present invention relates to a pharmaceutical composition comprising trametinib or a pharmaceutically acceptable salt thereof as active ingredient, at least one further pharmaceutical excipient and a carrier. The invention further relates to a composition for the preparation of said pharmaceutical composition and a method of preparing said pharmaceutical composition or said composition.

Trametinib has the chemical name N-{3-[3-cyclopropyl-5-(2-fluoro-4-iodophenylamino)-6,8-dimethyl-2,4,7-trioxo-3,4,6,7-tetrahydro-2H-pyrido[4,3-d]pyrimidin-1-yl]phenyl}acetamide represented by the following structural formula:

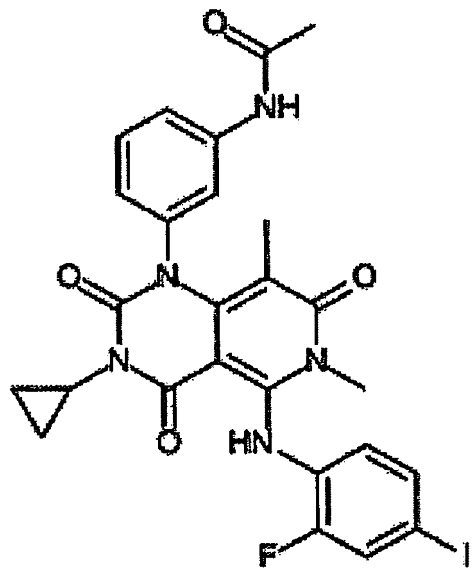

Trametinib can be prepared as described in US 2006/0014768. Trametinib is the compound of example 4-1 of this document. From this document it is also known that trametinib along with its pharmaceutically acceptable salts is useful as an inhibitor of MEK activity, particularly in treatment of cancer. As active ingredient in pharmaceutical preparations trametinib is used in its solvated form, in particular as dimethyl sulfoxide (DMSO) solvate. Tablets comprising trametinib DMSO solvate are described in WO 2012/088033, US 2012/0322817, US 2012/0283278 and US 2013/0109705.

WO 2012/088033 also teaches that trametinib in its non-solvated form is much less soluble than trametinib DMSO solvate which is said to negatively impact its pharmacodynamics when released from a pharmaceutical composition. It is also taught that there is a tendency of the DMSO solvate to revert to an insoluble desolvated form when exposed to moisture during the formulation process and that trametinib DMSO solvate can suffer from photo-instability.

Also the marketed formulation of this active ingredient includes the DMSO solvate form of trametinib to provide an adequate dissolution profile. However, the FDA approval package states that the marketed formulation Mekinist® is only approved as stable for 12 months for the 0.5 mg and 2 mg dosage form and 9 months for the 1 mg dosage form, respectively. Also, this is only valid when stored refrigerated between 2 and 8°C and protected from moisture and light. A further problem is that the active ingredient itself contains dimethyl sulfoxide which is classified as solvent class III by the European Pharmacopoeia. Solvents class III should be limited as hardly any long-term toxicity or carcinogenicity studies are available.

There is therefore still a need for further formulations for the active ingredient trametinib which do not suffer the above problems. In particular, a formulation for trametinib should exhibit adequate dissolution properties, should limit the exposure of patients to organic solvents, and in particular DMSO, should be stable enough to be stored at ambient conditions, preferably avoiding the requirement of desiccants in the packages, and should be prepared easily in a simple way thereby ensuring good content uniformity.

It has now surprisingly been found that the above problems can be solved by providing a pharmaceutical composition comprising the active ingredient trametinib in its non-DMSO solvated form and a carrier, if the carrier forms a matrix wherein the active ingredient is embedded. The present invention therefore relates to a pharmaceutical composition comprising trametinib or a pharmaceutically acceptable salt thereof as active ingredient, at least one further pharmaceutical excipient and a carrier, characterized in that the carrier forms a matrix wherein the active ingredient is embedded and the active ingredient contains less than 1 % w/w of dimethyl sulfoxide based on the total weight of trametinib in its salt free and non-solvate form present in the composition.

The present invention is based on the surprising finding that against the prior art teaching trametinib can be formulated in a pharmaceutical composition using the active ingredient in its non-DMSO solvated form instead of a DMSO solvate as used in the prior art compositions and the marketed Mekinist® tablets. Despite the teaching of WO 2012/088033, trametinib in its non-DMSO solvated from was found to exhibit adequate dissolution properties when formulated into a pharmaceutical composition together with a carrier embedding the active ingredient. Furthermore, it was found that a pharmaceutical composition comprising trametinib in its non-DMSO solvated form and a carrier can be stored at ambient conditions for a prolonged time without the requirement of any desiccant in the package. The pharmaceutical composition of the present invention shows dissolution properties being comparable to formulations containing trametinib DMSO solvate but at the same time shows improved storage stability and shelf-life. Furthermore, the undesired exposure of patients to organic solvents and in particular DMSO is avoided.

The active ingredient in the pharmaceutical composition of the present invention contains less than 1 % w/w of DMSO. Preferably, the active ingredient contains less than 0.8 % w/w, more preferably less than 0.6 % w/w, even more preferably less than 0.4 % w/w, such as less than 0.2 % w/w or less than 0.1 % w/w of DMSO. Most preferably, the active ingredient is substantially free of DMSO and even more preferably the active ingredient is free of DMSO. In this respect, all amounts in % w/w are based on the total weight of trametinib in its salt free and non-solvate form present in the composition.

In a further embodiment the pharmaceutical composition contains less than 1 % w/w, preferably less than 0.8 % w/w, more preferably less than 0.6 % w/w, even more preferably less than 0.4 % w/w, such as less than 0.2 % w/w or less than 0.1 % w/w of DMSO. Most preferably, the pharmaceutical composition is substantially free of DMSO and even more preferably the pharmaceutical composition is free of DMSO. In this respect, all amounts in % w/w are based on the total weight of trametinib in its salt free and non-solvate form present in the composition.

Nevertheless, the active ingredient, trametinib or a pharmaceutically acceptable salt thereof, may be present in the pharmaceutical composition of the present invention in the form of a solvate being different to the DMSO solvate. For example, the active ingredient can be present in the form of a hydrate or in the form of a solvate with any organic solvent molecule being different to DMSO. The organic solvent molecule may for example be an alcohol (such as methanol, ethanol, isopropanol, butanol or pentanol), acetone, ethylacetate, heptane or dichloromethane. Also mixed solvates such as a methanol/dichloromethane solvate is possible. In one embodiment of the present invention the active ingredient is present in a substantially non-solvated form, preferably in non-solvated form.

In the pharmaceutical composition of the present invention trametinib can be present in its salt free form or as a pharmaceutically acceptable salt thereof. Suitable pharmaceutical acceptable salts of trametinib are known to the person skilled in the art and are, for example, described in US 2006/0014768. Preferably, trametinib is present in its salt free form.

The carrier is present in the pharmaceutical composition in an amount sufficient to embed the active ingredient. Preferably, the carrier forms a matrix, in particular a solid matrix surrounding the active ingredient. This matrix can surround either particles of the active ingredient (thereby forming a solid dispersion) or single molecules of the active ingredient in which case the active ingredient is present in the polymer in the form of a solid solution.

The term "solid solution" is to be understood in the context of this invention as meaning that the active ingredient is distributed in a molecularly dispersed manner in a matrix which is present in a solid state at 20°C and a pressure of 101 kPa.

It is preferable that the solid solution of the invention contains less than 20 % w/w, more preferably less than 15 % w/w of active ingredient particles having a particle size of more than 300 nm, based on the total weight of the active ingredient present in the solid solution. More preferred, the solid solution contains substantially no, in particular no particles of active ingredient. In particular, the solid solution contains less than 20 % w/w, more preferably less than 15 % w/w and in particular no crystalline active ingredient of any crystal or crystallite size, based on the total weight of the active ingredient in the solid solution. The crystalline proportion can be determined by means of quantitative X-ray diffractometry according to the method of Hermans and Weidinger.

Thus, in a solid solution the active ingredient is dispersed on a molecular level in the polymer matrix. In this context, "molecularly dispersed" is to be understood as meaning that the solid solution contains substantially no, preferably less than 15, 10, 5 or 2 % w/w active ingredient particles with a particle size of more than 1 $\mu$m, preferably less than 15, 10, 5 or 2 % w/w of active ingredient particles with a particle size of more than 800 nm, preferably less than 15, 10, 5 or 2 % w/w of active ingredient particles with a particle size of more than 500 nm, preferably less than 15, 10, 5 or 2 % w/w of active ingredient particles with a particle size of more than 300 nm, more preferably less than 15, 10, 5 or 2 % w/w of active ingredient particles with a particle size of more than 200 nm, and most preferably less than 15, 10, 5 or 2 % w/w of active ingredient particles with a particle size of more than 100 nm, based on the total weight of the active ingredient in the solid solution. The particle size in this context may be determined by means of confocal Raman spectroscopy using an INTEGRA-Spectra Nanofinder of the company NT-MDT.

In a preferred embodiment, the solid solution is a "single-phase" solid solution. As a monophase system, the solid solution is defined by reference to a common glass transition point or the excipient and the active ingredient. This

can be analyzed by means of DSC.

As carrier, any pharmaceutically acceptable carrier may be used. Preferably the carrier is able to form a matrix embedding (surrounding) the active ingredient. More preferably, the carrier is able to form a solid solution comprising the active ingredient molecularly dispersed therein. In addition, the matrix or solid solution formed by the carrier may also include other substances like usual excipients known in the art. Finally, the matrix or solid solution may comprise one carrier or a mixture of two or more carriers.

The carrier used in the pharmaceutical composition of the present invention may be an inorganic compound or an organic compound. For example the carrier can be a polymer, which may be hydrophilic, amphiphilic or lipophilic, preferably hydrophilic or amphiphilic.

The carrier, in particular polymer that can be used for the preparation of the pharmaceutical composition preferably has a melting point (Ts) or a glass transition temperature (Tg) of 20°C or higher, preferably 20°C to 220°C, more preferably 20°C to 200°C, even more preferably 40°C to 180°C, most preferably 60°C to 140°C. By immobilisation, a polymer with a Tg selected accordingly is particularly advantageous in preventing the reformation of the molecular trametinib dispersion into colloids or particles.

The term "glass transition temperature" (Tg) is used to describe the temperature at which amorphous or partially crystalline polymers change from the solid state to the liquid state. In the process, a distinct change in physical parameters, e.g. hardness and elasticity, occurs. Below the Tg, a polymer is usually glassy and hard, whereas above the Tg, it changes into a rubber-like to viscous state. The glass transition temperature is determined in the context of this invention by means of dynamic differential scanning calorimetry (DSC). For this purpose a Mettler Toledo DSC 1 apparatus can be used. The work is performed at a heating rate of 10°C/min, and at a cooling rate of 15°C/min.

In addition, the polymer preferably has a number-average molecular weight of 1,000 to 4,000,000 g/mol, more preferably from 1,000 to 3,500,000 g/mol, even more preferably from 1,500 to 3,000,000 g/mol, and particularly preferably from 3,500 to 2,500,000 g/mol. When the polymer is dissolved in (distilled) water (if the polymer is insoluble in water any other suitable solvent as described in "Handbook of Pharmaceutical Excipients" Sixth Edition, Edited by Raymond C Rowe, Paul J Sheskey and Marianne E Quinn, Pharmaceutical Press and American Pharmacists Association 2009 is to be used) in an amount of 2 % w/w, the resulting solution preferably has a viscosity of <150,000 mPa•s, more preferably <100,000 mPa•s, especially <75,000 mPa•s, measured at 25°C. The viscosity is measured in accordance with the European Pharmacopoeia (Ph. Eur.), 6th edition, section 2.2.10.

Hydrophilic and amphiphilic polymers are preferably used. "Hydrophilic" refers to polymers which possess hydrophilic groups. Examples of suitable hydrophilic groups are hydroxy, alkoxy, acrylate, methacrylate, sulphonate, carboxylate and quaternary ammonium groups. Hydroxy groups are preferable.

Suitable polymers are for example cellulosic polymers and non-cellulosic polymers, such as methyl cellulose (MC), ethyl cellulose (EC), hydroxyethyl cellulose (HEC), hydroxyethyl methyl cellulose (HEMC), hydroxypropyl cellulose (HPC), hydroxypropyl methyl cellulose (HPMC), hydroxypropyl methyl cellulose phthalate (HPMCP), hydroxypropyl methyl cellulose acetate (HPMCA), hydroxypropyl methyl cellulose acetate succinate (HPMCAS), hydroxypropyl cellulose acetate succinate (HPCAS), hydroxyethyl cellulose acetate succinate (HECAS), hydroxyethyl methyl cellulose succinate (HEMCS), hydroxyethyl methyl cellulose acetate succinate (HEMCAS), carboxymethyl cellulose (CMC), carboxyethyl cellulose (CEC), carboxymethyl ethyl cellulose (CMEC), hydroxypropyl methyl cellulose acetate phthalate (HPMCAP), hydroxypropyl methyl cellulose acetate trimellitate (HPMCAT), polyvinyl alcohol (PVA), polyvinyl pyrrolidone (PVP), polyvinyl acetate (PVAc), vinylpyrrolidon vinylacetat copolymer (VP/VAc copolymer, such as Copovidon Kollidon VA 64), polyvinylacetat polyvinylpyrrolidon copolymer (PVAc/PVP copolymer, Povidon), polyvinyl polypyrrolidon (PVPP copolymer, Crospovidon), polyvinyl alcohol polyethylene glycol copolymer (PVA/PEG copolymer), poly propylen glycols (PPGs), poly ethylen glycols (PEGs 1500, 3000, 3350, 4000, 6000, 8000, 10000, 12000, 20000), polyoxyethylene polyoxypropylene copolymers (Poloxamers), polymethacrylates (such as the EUDRAGITS®), carboxylic acid functionalized polyacrylates (such as the EUDRAGITS®), amine-functionalized polyacrylates and polymethacrylates, acrylate and methacrylate copolymers (such as the EUDRAGITS®), and polyoxyethylene-polyoxypropylene copolymers (such as the PLURONICS®), or mixture thereof.

The pharmaceutical composition of the invention may in particular comprise the following polymers: polyvinyl pyrrolidone, polyvinyl acetate (PVAC), polyvinyl alcohol (PVA), polymers of acrylic acid and their salts, polyacrylamide, polymethacrylates, vinyl pyrrolidone/vinyl acetate copolymers (such as Kollidon® VA64, BASF), polyalkylene glycols, such as polypropylene glycol or preferably polyethylene glycol, co-block polymers of polyethylene glycol, especially co-block polymers of polyethylene glycol and polypropylene glycol (Pluronic®, BASF), polyethylene oxide, hydroxypropyl methyl cellulose (HPMC), hydroxypropyl methyl cellulose acetate succinate (HPMCAS), polyvinylcaprolactam-polyvinylacetate-polytethylene glycol graft copolymer (Soluplus®) and mixtures thereof.

Further examples of possible polymers comprise: polysaccharides, such as hydroxypropyl methyl cellulose (HPMC), hydroxypropyl methyl cellulose acetate succinate (HPMCAS), carboxymethyl cellulose (CMC, especially sodium and calcium salts), ethyl cellulose, methyl cellulose, hydroxyethyl cellulose, ethyl hydroxyethyl cellulose, hydroxypropyl cellulose (HPC); microcrystalline cellulose, and mixtures of the polymers mentioned; or mixtures of the polymers mentioned with polymers listed above.

Apart from that, natural gum can be used as the polymer, e.g. gum traganth, alginates, gum Arabic and gum guar.

Furthermore, the matrix or solid solution may also include solid, non-polymeric compounds which preferably contain polar side groups. Examples of these are sugar alcohols and sugars. Examples of sugar alcohols are glycerol, xylitol, sorbitol, mannitol, isomalt and maltitol. Examples of sugars are aldoses, such as glucose and galactose, ketoses such as fructose, acetales, such as saccarose and trehalose, and hemiacetales, such as maltose and lactose.

In addition, the matrix or solid solution may also include substances which behave like polymers, in particular semisolid excipients. Examples of these are fats and waxes. It is, for example, possible to use waxes, such as cetyl palmitate, carnauba wax or bees' wax. It is likewise possible to use fats, such as glycerol fatty acid esters (e.g. glycerol palmitate, glycerol behenate, glycerollaurate, glycerol stearate), PEG glycerol fatty acid esters or vegetable oils or hydrogenated vegetable oils. Further examples are glycerol, stearyl alcohol, salts of fatty acids (e.g. aluminum monostearate). Further specific examples of semisolid excipients are PEG 400, poly(oxy-1,2-ethanediyl) (Solutol HS 15), poloxamer 407 (Pluronic F127), macrogolglycerolhydroxystearate 40 (Cremophor RH 40), ceteareth - 6 and stearyl alcohol (Cremophor A6), lauroyl macrogol-32 glycerides (Gelucire 44/14), stearoyl macrogol-32 glycerides (Gelucire 50/13) and macrogol-20-glycerolmono-stearat (Tagat S2).

It is preferable that the type and quantity of the carrier should be selected such that the resulting matrix or solid solution has a glass transition temperature (Tg) of 20°C or higher, preferably of 25°C or higher.

The present invention furthermore relates to intermediates which are useful in the preparation of the above described pharmaceutical composition. The term "intermediate" is to be understood as a composition comprising the ingredients as defined below. The terms "intermediate" and "composition" are used interchangeably herein. Such composition may be used in the preparation of a pharmaceutical composition. However, the "intermediate" as such is not a pharmaceutical composition (although it may be suitable as pharmaceutical composition). For example, the intermediate may be in the form of granules which upon compression into tablets are prepared into a pharmaceutical composition.

The intermediates according to the invention comprise trametinib or a pharmaceutically acceptable salt thereof as active ingredient and a carrier wherein the carrier forms a matrix wherein the active ingredient is embedded and the active ingredient, preferably the intermediate, contains less than 1 % w/w of DMSO, preferably less than 0.8 % w/w of DMSO, more preferably less than 0.6 % w/w of DMSO, even more preferably less than 0.4 % w/w of DMSO, such as less than 0.2 % w/w or less than 0.1 % w/w of DMSO, each based on the total weight of trametinib in its salt free and non-solvate form present in the composition. In a further preferred embodiment the intermediate is substantially free of DMSO, in particular free of DMSO.

Preferably, the intermediate has a glass transition temperature (Tg) of 20°C or higher, preferably 25°C or higher.

Preferably, the intermediate contains one or more of the above described carriers.

In a further embodiment, the intermediate of the invention contains the active ingredient in an amount such that the weight ratio of active ingredient to carrier is in the range of 1:1 to 1:150, preferably in the range of 1:1 to 1:100.

The pharmaceutical composition of the present invention may comprise said intermediate and at least one further pharmaceutical excipient.

The pharmaceutical compositions and intermediates of the invention are obtainable by a variety of preparation methods. Depending on the preparation method, the products are obtained in different particle sizes. Normally, the products and in particular intermediates of the invention are present in particulate form and have an average particle diameter (D50) of 1 to 750 μm, depending on the preparation method.

The expression "average particle diameter" relates in the context of this invention to the D50 value of the volume average particle diameter determined by means of laser diffractometry. In particular, a Malvern Instruments Mastersizer 2000 was used to determine the diameter (wet measurement with ultrasound for 5 min., 2,500 rpm, the evaluation using the Fraunhofer method, and preferably using sun flower oil as dispersant). The average particle diameter, which is also referred to as the D50 value of the integral volume distribution, is defined in the context of this invention as the particle diameter at which 50 % by volume of the particles have a smaller diameter than the diameter which corresponds to the D50 value. Similarly, 50 % by volume of the particles then have a larger diameter than the D50 value.

Another subject matter of the invention is a method of preparing the pharmaceutical composition or the intermediate of the invention. In the following, two preferred embodiments of such a method will be explained.

In a first preferred embodiment, the invention relates to a drying, in particular spray-drying or freeze-drying method comprising the steps of

(a1) dissolving the active ingredient and the carrier in a solvent, and
(b1) drying, preferably spray-drying or freeze-drying the solution obtained in step (a1).

In step (a1), trametinib and the carrier described above, are dissolved, preferably completely dissolved, in a solvent or mixture of solvents. Crystalline or amorphous trametinib may be used.

Suitable solvents are, for example, water, alcohol (e.g. methanol, ethanol, isopropanol, butanol, pentanol),

dimethyl sulphoxide (DMSO), acetone, ethyl acetate, heptane, dichloromethane or mixtures thereof. Preferably, a methanol/dichloromethane mixture is used.

In the subsequent step (b1), the solution from step (a1) is dried, preferably spray-dried or freeze-dried. The spray-drying is usually carried out in a spray tower. As an example, a Büchi B-290 is suitable (Büchi Labortechnik GmbH, Germany). Preferably, an inlet temperature of 60°C to 160°C is chosen. The aspirator preferably runs at 80 to 100 %. Spray-drying has the advantage of a continuous method, which enhances the reproducibility and hence also the homogeneity and uniformity of content of active agent. Generally, the freeze-drying process comprises two stages. Stage 1: Freezing the solution and reducing the pressure, preferably below the triple point of the solution. Stage 2: Raising the temperature, preferably to the sublimation curve, in order to allow latent heat of sublimation. After the sublimation is complete, the freeze-dried (lyophilised) substrate is warmed to room temperature.

The process conditions in this first embodiment are preferably selected such that the resulting intermediate particles have a volume-average particle diameter (D50) of 1 to 250 μm, more preferably 2 to 150 μm, especially 3 to 100 μm.

In a second preferred embodiment, the invention relates to a melt process, preferably a melt extrusion process, i.e. a method comprising the steps of

(a2) mixing the active ingredient and the molten carrier, and
(b2) extruding the mixture obtained in step a(2).

In step (a2), trametinib is mixed with the carrier, preferably in a mixer. Crystalline or amorphous trametinib may be used.

Suitable carriers in this embodiment are especially polyvinyl pyrrolidone and vinyl pyrrolidone/vinyl acetate copolymers, and also polyvinyl alcohols, methacrylates, HPMC, HPMCAS and polyvinyl caprolactam-vinylacetate-polyethylene glycol graft copolymer.

The mixture from step (a2) is conventionally processed in the extruder into a homogeneous melt. In step (b2), the mixture is extruded.

Conventional melt extruders can be used as the extruders. The screw profile of the extruder preferably contains kneading units. The shear forces created in this way contribute to melting the mixture and thus to dissolving the active agent in the matrix material. By way of example, a Leistritz Micro 18 is used.

The extrusion temperature depends on the nature of the polymer. It usually lies between 50 and 250°C, preferably between 60 and 200°C, more preferably between 80 and 180°C. The extrusion is preferably carried out at an outlet pressure of 2 bar to 100 bar, more preferably at 5 to 80 bar.

The cooled melt is usually comminuted by a rasp screen (e.g. Comil® U5) and in this way reduced to a uniform particle size.

The process conditions in this second embodiment are preferably selected such that the resulting intermediate particles have a volume-average particle diameter (D50) of 150 to 1,000 μm, more preferably a D50 of 250 to 800 μm.

Instead of granulating the extruded material, "direct injection moulding" may also be performed. In this case, the method of the invention includes the step of

(c2) injection moulding the extruded material into moulds for pharmaceutical dosage forms.

Examples are moulds for tablets.

Of the preparation methods described, the first embodiment is particularly preferable. It, too, permits a continuous process, which improves the reproducibility of the method as a whole, and hence also the uniformity of content of active agent in the intermediate and products prepared from it.

The intermediate of the invention (e.g. the molecularly dispersed trametinib of the invention) is usually employed to prepare a pharmaceutical formulation.

The invention therefore further relates to a pharmaceutical composition comprising said intermediate and at least one further pharmaceutical excipient.

The pharmaceutical composition may be present as solid oral dosage form, for example, in the form of a granulate, capsule or tablet. Tablets are preferred. It is also preferable that the pharmaceutical composition is intended for oral administration, especially for peroral administration (for swallowing).

The further pharmaceutical excipients are excipients with which the person skilled in the art is familiar, such as those which are described in the European Pharmacopoeia.

Examples of further pharmaceutical excipients used are disintegrants, anti-stick agents, emulsifiers, pseudo-emulsifiers, fillers, additives to improve the powder flowability, glidants, wetting agents, gelling agents and/or lubricants. Where appropriate, further excipients can also be used.

The ratio of active agent to excipients is preferably selected such that the resulting pharmaceutical compositions

contain 0.1 to 4 % w/w, more preferably 0.12 to 2.5 % w/w, especially 0.12 to 1.75 % w/w, more preferably 0.15 to 1.5 % w/w of trametinib, each based on the total weight of the pharmaceutical composition.

In these amounts, the amount of carrier used to prepare the intermediate of the invention is counted as an excipient. This means that the amount of active agent refers to the amount of trametinib (in its salt free and solvate free form) contained in the formulation.

The intermediate preferably accounts for 1 to 40 % w/w of the total weight of the composition, more preferably 2 to 35 % w/w, even more preferably 5 to 35 % w/w and especially 7 to 35 % w/w, based on the total weight of the pharmaceutical composition.

It has been shown that the intermediates of the invention are suitable for serving as a basis for a dosage form with immediate release (or "IR" for short).

In a preferred embodiment for an IR formulation, a disintegrant is used. In that preferred embodiment, the pharmaceutical composition of the invention therefore contains

(i) 1 to 40 % w/w, more preferably 5 to 35 % w/w, especially 7 to 35 % w/w intermediate and
(ii) 1 to 30 % w/w, more preferably 2 to 15 % w/w, especially 4 to 10 % w/w disintegrants, based on the total weight of the composition.

"Disintegrants" is the term generally used for substances which accelerate the disintegration of a dosage form, especially a tablet, after it is placed in water. Suitable disintegrants are, for example, organic disintegrants such as carrageenan, croscarmellose (including croscarmellose sodium), sodium carboxymethyl cellulose, sodium carboxymethyl starch and crospovidone. Alkaline disintegrants are likewise used. The term "alkaline disintegrants" means disintegrants which, when dissolved in water, produce a pH level of more than 7.0.

It is also possible to use inorganic alkaline disintegrants, especially salts of alkali and alkaline earth metals. Preferred examples here are sodium, potassium, magnesium and calcium. As anions, carbonate, hydrogen carbonate, phosphate, hydrogen phosphate and dihydrogen phosphate are preferred. Examples are sodium hydrogen carbonate, sodium hydrogen phosphate, calcium hydrogen carbonate and the like.

Sodium carboxymethyl starch or sodium carboxymethyl cellulose, particularly preferably sodium carboxymethyl starch, are particularly preferably used as disintegrants, especially in the above-mentioned amounts.

The composition of the invention may contain fillers. "Fillers" generally means substances which serve to form the body of the tablet in the case of tablets with small amounts of active agent (e.g. less than 70 % w/w). This means that fillers "dilute" the active agents in order to produce an adequate tablet-compression mixture. The normal purpose of fillers, therefore, is to obtain a suitable tablet size.

Examples of preferred fillers are lactose, lactose derivatives, mannitol, starch, starch derivatives, microcrystalline cellulose, treated starch, talcum, calcium phosphate, sucrose, calcium carbonate, magnesium carbonate, magnesium oxide, maltodextrin, calcium sulphate, dextrates, dextrin, dextrose, hydrogenated vegetable oil, kaolin, sodium chloride, and/or potassium chloride. Prosolv® (Rettenmaier & Söhne, Germany) can likewise be used.

Fillers are normally used in an amount of 1 to 99 % w/w, more preferably 30 to 95 % w/w, based on the total weight of the composition. In addition, it is, for example, possible for at least 40 % w/w or at least 50 % w/w filler to be used.

One example of an additive to improve the powder flowability is disperse or colloidal silica, e.g. known under the trade name Aerosil®.

Additives to improve the powder flowability (glidants) are generally used in an amount of 0.1 to 3 % w/w, based on the total weight of the formulation.

In addition, lubricants may be used. Lubricants are generally used in order to reduce sliding friction. In particular the intention is to reduce the sliding friction found during tablet pressing between the punch moving up and down in the die and the die wall, on the one hand, and between the edge of the tablet and the die wall, on the other hand. Suitable lubricants are, for example, stearic acid, adipic acid, sodium stearyl fumarate (Pruv®) and/or magnesium stearate.

Lubricants are generally used in an amount of 0.1 to 3 % w/w, based on the total weight of the formulation.

The pharmaceutical composition of the invention is preferably compressed into tablets.

The intermediates of the invention can therefore be compressed into tablets by means of direct compression or are subjected to dry granulation before being compressed into tablets. Intermediates with a bulk density of less than 0.5 g/ml are preferably processed by dry granulation.

Direct compression is especially preferred if the intermediate is prepared by means of melt extrusion (process steps (a2) and (b2)).

Dry granulation is especially preferred if the intermediate is prepared by means of spray-drying (process steps (a1) and (b1)).

In a further aspect, the present invention therefore relates to a dry granulation process, i.e. a method of preparing granules, comprising the steps of

(I) preparing the intermediate of the invention and one or more pharmaceutical excipients (especially those described above);
(II) compacting it into flakes; and
(III) granulating or comminuting the flakes into granules.

In step (I), trametinib in the form of the solid solution (i.e. in the form of the intermediate of the invention) and excipients are preferably mixed. The mixing can be performed in conventional mixers. Alternatively, it is possible that the trametinib is initially only mixed with part of the excipients (e.g. 50 to 95 %) before compacting (II), and that the remaining part of the excipients is added after the granulation step (III). In the case of multiple compacting, the excipients should preferably be mixed in before the first compacting step, between multiple compacting steps or after the last granulation step.
In step (II) of this method, the mixture from step (I) is compacted into flakes. It is preferable here that it should be dry compacting, i.e. the compacting is preferably performed in the absence of solvents, especially in the absence of organic solvents.
The compacting conditions are, for example, selected such that the intermediate of the invention is present in the form of compacted material (flakes), the density of the intermediate (or the flakes) being 0.8 to 1.3 g/$cm^3$, preferably 0.9 to 1.20 g/$cm^3$, especially 1.01 to 1.15 g/$cm^3$.
The term "density" here preferably relates to the "pure density" (i.e. not to the bulk density or tapped density). The pure density can be determined with a gas pycnometer. The gas pycnometer is preferably a helium pycnometer; in particular, the AccuPyc 1340 helium pycnometer from the manufacturer Micromeritics, Germany, is used.
The compacting is preferably carried out in a roller compactor.
The rolling force is preferably 5 to 70 kN/cm, preferably 10 to 60 kN/cm, more preferably 15 to 50 kN/cm.
The gap width of the roll granulator is, for example, 0.8 to 5 mm, preferably 1 to 4 mm, more preferably 1.5 to 3 mm, especially 1.8 to 2.8 mm.
The compacting apparatus used preferably has a cooling means. In particular, the cooling is preferably such that the temperature of the compacted material does not exceed 50°C, especially 40°C.
In step (III) of the method, the flakes are granulated, or comminuted into granules. The granulation can be performed with methods known in the prior art.
In a preferred embodiment, the granulation conditions are selected such that the resulting particles (granules) have a volume-average particle size (D50) value) of 50 to 800 μm, more preferably 100 to 750 μm, even more preferably 150 to 500 μm, especially 200 to 450 μm.
In a preferred embodiment, the granulation is performed in a screen mill. In this case, the mesh width of the screen insert is usually 0.1 to 5 mm, preferably 0.5 to 3 mm, more preferably 0.75 to 2 mm, especially 0.8 to 1.8 mm.
In a preferred embodiment, the method is adapted such that multiple compacting occurs, with the granules resulting from step (III) being returned once or more times to the compacting (II). The granules from step (III) are preferably returned 1 to 5 times, especially 2 to 3 times.
The granules resulting from step (IV) can be further used or processed into pharmaceutical dosage forms. For this purpose, the granules are filled into sachets or capsules. The granules resulting from step (III) are, however, preferably compressed into tablets (=step 1V).
This means that a further subject matter of the invention is a method of preparing a tablet, comprising the process of preparing granules, and further comprising the following step:

(IV) compressing the granules, and optionally one or more additional pharmaceutical excipients, into a tablet.

In step (IV) of the method, the granules obtained in step (III) are compressed into tablets, i.e. the step involves compression into tablets. Compression can be performed with tableting machines known in the prior art.
In step (IV) of the method, pharmaceutical excipients may optionally be added to the granules from step (III).
The amounts of excipients which may be added in step (IV) usually depend on the type of tablet to be produced and the amount of excipients which were already added in steps (I) or (II). This preferably involves the addition or one or more lubricants, such as those already described above.
In the case of direct compression, only steps (I) and (IV) of the method described above are performed. This means that a further subject matter of the invention is a method of preparing a tablet, comprising the following steps:

(I) preparing, and optionally mixing, the intermediate of the invention and one or more pharmaceutical excipients (especially those described above);
(IV) compressing the intermediate and the one or more pharmaceutical excipients into a tablet.

The method preferably does not involve any further steps between these two steps.

The tableting conditions are preferably selected such that the resulting tablets have a tablet height to weight ratio of 0.005 to 0.030 mm/mg, more preferably 0.006 to 0.030 mm/mg, particularly preferably 0.015 to 0.025 mm/mg.

In accordance with the invention, the resulting tablets preferably have a mass of 70 to 550 mg, such as 90 to 350 mg or particularly preferably 110 to 260 mg.

In addition, the resulting tablets preferably have a hardness of 50 to 200 N, particularly preferably 50 to 150 N. The hardness is determined in accordance with Ph. Eur. 6.0, section 2.9.8.

In addition, the resulting tablets preferably have a friability of less than 5 %, particularly preferably less than 3 %, especially less than 2 %. The friability is determined in accordance with Ph. Eur. 6.0. section 2.9.7.

Finally, the tablets of the invention usually exhibit a content uniformity of trametinib, determined in accordance with Ph. Eur. 2.9.6, which is characterised in that each of the dosage form units has a content of trametinib which lies between 90 and 110 %, preferably 95 to 105 %, especially 98 to 102 % of the average content of those ten dosage form units.

In particularly preferred embodiments, trametinib (in its non-solvated and salt free form) is contained in the composition in amounts of about 0.5 mg, about 1.0 mg or about 2.0 mg.

In the case of an IR formulation, the release profile of the tablets of the invention according to the USP method (USP paddle apparatus II, 1,000 ml test medium; 50 mM $KH_2PO_4$ buffer pH 6.8, 37°C and 75 rpm) after 10 minutes usually indicates a content released of at least 30 %, preferably at least 60 %, especially at least 90 %.

The above details regarding hardness, friability, content uniformity and release profile preferably relate here to the non-film-coated tablet.

The tablets produced by the method of the invention are preferably tablets for oral administration and specifically ones which can be swallowed unchewed (non-film-coated or preferably film-coated).

In the case of tablets which are swallowed unchewed, it is preferable that they be coated with a film layer. For this purpose, the methods of film-coating tablets which are standard in the prior art may be employed. The abovementioned ratios of active agent to excipient, however, relate to the non-film-coated tablet.

For film-coating, macromolecular substances are preferably used, such as modified celluloses, polymethacrylates, polyvinyl pyrrolidone, polyvinyl acetate phthalate, zein and/or shellac or natural gum, such as carrageenan.

The thickness of the coating is preferably 1 to 100 $\mu$m.

In the attached figures

Figure 1 shows the dissolution profile of Mekinist® tablets (Comparative Example 1),
Figure 2 shows the dissolution profile of the tablets according to Example 1,
Figure 3 shows the dissolution profile of the tablets according to Example 2,
Figure 4 shows the dissolution profile of the tablets according to Example 3, and
Figure 5 shows the dissolution profile of the tablets according to Example 4.

The invention will now be illustrated with reference to the following examples.

EXAMPLES

In the following examples the dissolution profiles of the obtained tablets were measured (photometric determination at 330 nm) as described in USP, apparatus II, Paddle Method using 1,000 ml dissolution medium (50 mM $KH_2PO_4$ buffer; pH 6.8) at 75 rpm and a temperature of 37°C.

Comparative example 1

In comparative example 1 Mekinist® tablets 2 mg as marketed in the US were tested. These tablets contain trametinib dimethyl sulfoxide solvate as active ingredient. According to the prescribing information the inactive ingredients of Mekinist® tablets are mannitol, microcrystalline cellulose, hypromellose, croscarmellose sodium, magnesium stearate, sodium lauryl sulphate and colloidal silicon dioxide in the tablet core. Hypromellose, titanium dioxide, polyethylene glycol, polysorbate 80 and iron oxide red are used in the coating of the 2 mg tablets. The dissolution profile of Mekinist 2 mg tablets as measured according to the above method is shown in attached figure 1.

**Example 1**

Tablets comprising the following ingredients were prepared:

| | Ingredient | Amount [mg] | Percentage |
|---|---|---|---|
| 1 | Trametinib, non-DMSO solvated | 2.00 | 1.25 |
| 2 | Copovidone (Kollidon VA64) | 18.00 | 11.25 |
| 3 | Methanol | | - |
| 4 | Dichlormethane | | - |
| 5 | Prosolv Easytab (ready-to-use mix of MCC, sodium starch glycolate, sodium stearyl fumarate and silica) | 132.00 | 82.50 |
| 6 | Croscarmellose sodium (Ac-Di-Sol) | 8.00 | 5.00 |
| | | | |
| | total | 160.00 | 100.0 |

Manufacturing:

Copovidone was dissolved under constant stirring in a mixture of methanol/dichlormethane. Trametinib was added and the solution was stirred until the trametinib was fully dissolved. The solution was spray dried with a Büchi B-290 under nitrogen atmosphere (inlet air temperature 90°C, aspirator 100 %, pump rate 6 %). The resulting powder was dried in a vacuum cabinet dryer at 40°C and 0.1 mbar for 66 hours.

The trametinib-polymer preparation (which according to the present invention can be understood as "intermediate") was mixed with ingredients 5 and 6 for 5 minutes, sieved (mesh size 500 μm), mixed for another 15 minutes and compressed to tablets of diameter of 8 mm.

The dissolution profile of the obtained tablets is shown in figure 2.

**Example 2**

Tablets comprising the following ingredients were prepared:

| | Ingredient | Amount [mg] | Percentage |
|---|---|---|---|
| 1 | Trametinib, non-DMSO solvated | 2.00 | 0.91 |
| 2 | HPMCAS (Aqoat AS-LG) | 18.00 | 8.18 |
| 3 | Methanol | | - |
| 4 | Dichlormethane | | - |
| 5 | Prosolv Easytab (ready-to-use mix of MCC, sodium starch glycolate, sodium stearyl fumarate and silica) | 187.90 | 85.41 |
| 6 | Croscarmellose sodium (Ac-Di-Sol) | 11.00 | 5.00 |
| 7 | AEROSIL 200 | 1.10 | 0.50 |
| | total | 220.00 | 100.0 |

Manufacturing:

HPMCAS (hydroxypropyl methylcellulose acetate succinate) was dissolved under constant stirring in a mixture of methanol/dichlormethane. Trametinib was added and the solution was stirred until the trametinib was fully dissolved. The solution was spray dried with a Büchi B-290 under nitrogen atmosphere (inlet air temperature 90°C, aspirator 100 %, pump rate 6 %). The resulting powder was dried in a vacuum cabinet dryer at 40°C and 0.1 mbar for 66 hours.

The trametinib-polymer preparation was mixed with ingredients 5, 6 and 7 for 5 minutes, sieved (mesh size 500 μm), mixed for another 15 minutes and compressed to tablets of diameter of 8 mm.

The dissolution profile of the obtained tablets is shown in figure 3.

**Example 3**

Tablets comprising the following ingredients were prepared:

| | Ingredient | Amount [mg] | Percentage |
|---|---|---|---|
| 1 | Trametinib, non-DMSO solvated | 2.00 | 1.21 |
| 2 | Copovidone (Kollidon VA64) | 17.00 | 10.30 |
| 3 | Sodium dodecyl sulfate | 1.00 | 0.61 |
| 4 | Methanol | | - |
| 5 | Dichlormethane | | - |
| 6 | Prosolv Easytab (ready-to-use mix of MCC, sodium starch glycolate, sodium stearyl fumarate and silica) | 135.92 | 82.38 |
| 7 | Croscarmellose sodium (Ac-Di-Sol) | 8.25 | 5.00 |
| 8 | AEROSIL 200 | 0.83 | 0.50 |
| | total | 165.00 | 100.0 |

Manufacturing:

Copovidone and SDS were dissolved under constant stirring in a mixture of methanol/dichlormethane. Trametinib was added and the solution was stirred until the trametinib was fully dissolved. The solution was spray dried with a Büchi B-290 under nitrogen atmosphere (inlet air temperature 90°C, aspirator 100 %, pump rate 6 %). The resulting powder was dried in a vacuum cabinet dryer at 40°C and 0.1 mbar for 16 hours.

The trametinib-polymer preparation was mixed with ingredients 6 - 8 for 5 minutes, sieved (mesh size 500 μm), mixed for another 15 minutes and compressed to tablets of diameter of 8 mm.

The dissolution profile of the obtained tablets is shown in figure 4.

**Example 4**

Tablets comprising the following ingredients were prepared:

| | Ingredient | Amount [mg] | Percentage |
|---|---|---|---|
| 1 | Trametinib, non-DMSO solvated | 2.00 | 0.91 |
| 2 | Copovidone (Kollidon VA64) | 17.00 | 7.73 |
| 3 | Isomalt, micronized (GalenIQ 721) | 20.00 | 9.09 |
| 4 | Sodium dodecyl sulfate | 1.00 | 0.45 |
| 5 | Methanol | | - |
| 6 | Dichlormethane | | - |
| 7 | Prosolv Easytab (ready-to-use mix of MCC, sodium starch glycolate, sodium stearyl fumarate and silica) | 167.00 | 75.91 |
| 8 | Croscarmellose sodium (Ac-Di-Sol) | 11.00 | 5.00 |
| 9 | Sodium dodecyl sulfate | 1.00 | 0.45 |
| 10 | Silica (AEROSIL 200) | 1.00 | 0.45 |
| | total | 220.00 | 100.0 |

Manufacturing:

Copovidone and SDS (4) were dissolved under constant stirring in a mixture of methanol/dichlormethane. Trametinib was added and the solution was stirred until the trametinib was fully dissolved. Isomalt was suspended and homogenized in the solution by means of an UltraTurrax. The suspension was spray dried with a Büchi B-290 under nitrogen atmosphere (inlet air temperature 85°C, aspirator 100 %, pump rate 6 %) under constant stirring. The resulting powder was dried in a vacuum cabinet dryer at 40°C and 0.1 mbar for 32 hours.

Ingredients 9 and 10 (pre-sieved 500) were mixed for 10 minutes. The trametinib-polymer preparation was mixed with ingredients 9 and 10 for another 10 minutes, sieved (mesh size 500 μm), mixed for another 10 minutes. Ingredients 7 and 8 were added and mixed for another 15 minutes. Tablets of diameter of 8 mm were compressed.

The dissolution profile of the obtained tablets is shown in figure 5.

**Example 5:**

Tablets comprising the following ingredients were prepared:

| | Ingredient | Amount [mg] | Percentage |
|---|---|---|---|
| 1 | Trametinib, non-DMSO solvated | 2.00 | 0.91 |
| 2 | Soluplus | 37.00 | 16.82 |
| 3 | Sodium dodecyl sulfate | 1.00 | 0.45 |
| 7 | Prosolv Easytab (ready-to-use mix of MCC, sodium starch glycolate, sodium stearyl fumarate and silica) | 167.00 | 75.91 |
| 8 | Croscarmellose sodium (Ac-Di-Sol) | 11.00 | 5.00 |
| 9 | Sodium dodecyl sulfate | 1.00 | 0.45 |
| 10 | Silica (AEROSIL 200) | 1.00 | 0.45 |
| | total | 220.00 | 100.0 |

Manufacturing:

Trametinib, Soluplus and SDS (3) were melted on a heating plate and stirred for homogenization purposes. The melt was cooled down to room temperature, ground and sieved (mesh size 500 μm).

Excipients 7 to 10 (pre-sieved 500 μm) were mixed for 10 minutes. The API-Polymer preparation was added, mixed for 10 minutes, sieved (mesh size 500 μm) and mixed for another 10 minutes. Tablets of diameter of 8 mm were compressed.

**Example 6:**

Bioavailability studies (beagle dog study) were conducted using the pharmaceutical compositions of Examples 2, 3 and 4 and Mekinist® tablets as reference. The results are summarized in the following table:

Group 1: Reference
Group 2: corresponds to example 3
Group 3: corresponds to example 4
Group 4: corresponds to example 2

| Pharmacokinetic parameters of Trametinib (non-compartmental analysis) (dosage: 2 mg Trametinib formulation/ animal) Mean values per group (n = 6) | | | | | | | | |
|---|---|---|---|---|---|---|---|---|
| **Group** | **Product** | **$C_{max}$## [ng/mL]** | **$t_{max}$## [h]** | **$t_{1/2}$ [h]** | **$K_{et}$ [1/h]** | **$AUC_{0-120\ h}$ [h·ng/mL]** | **$AUC_{0-inf}$ [h·ng/mL]** | **$F_{rel}$** |
| 1 | Trametinib DMSO (Mekinist®) | 65.0 | 3.17 | 32.0 | 0.022 | 1680 | 1804 | - |
| 2 | Trametinib nonsolvated | 65.9 | 2.67 | 34.2 | 0.021 | 1927 | 2075 | 115.0 |
| 3 | Trametinib nonsolvated | 106.9 | 0.83 | 32.9 | 0.022 | 2540 | 2739 | 151.9 |
| 4 | Trametinib nonsolvated | 91.4 | 1.75 | 31.7 | 0.022 | 1794 | 1923 | 106.6 |
| ##: Values obtained from the analytical results, all other values calculated by pharmacokinetic evaluation. | | | | | | | | |

$$F_{rel}: \quad \text{Relative bioavailability [\%]} = \frac{AUC_{0\text{-}inf}\ (\text{group 2, 3, or 4})}{AUC_{0\text{-}inf}\ (\text{group 1})} \times 100$$

## Claims

1. Pharmaceutical composition comprising trametinib or a pharmaceutically acceptable salt thereof as active ingredient, at least one further pharmaceutical excipient and a carrier, **characterized in that** the carrier forms a matrix wherein the active ingredient is embedded and the active ingredient contains less than 1 % w/w of dimethyl sulfoxide based on the total weight of trametinib in its salt free and non-solvate form present in the composition.

2. Pharmaceutical composition according to claim 1, wherein the composition contains less than 1 % w/w of dimethyl sulfoxide based on the total weight of trametinib in its salt free and non-solvate form present in the composition.

3. Pharmaceutical composition according to claim 1 or 2, wherein the carrier is selected from polymers, fats, waxes, sugars, sugar alcohols, glycol, fatty acids, salts of fatty acids, fatty acid esters and fatty alcohols.

4. Pharmaceutical composition according to any of the preceding claims, wherein the active ingredient is present in the carrier in the form of a solid solution or solid dispersion.

5. Pharmaceutical composition according to any of the preceding claims, wherein the carrier has a glass transition temperature (Tg) of 20°C or higher.

6. Pharmaceutical composition according to any of the preceding claims, wherein the carrier is hydrophilic or amphiphilic.

7. Pharmaceutical composition according to any of the preceding claims, wherein the carrier is selected from the group consisting of polyvinyl pyrrolidone, polyvinyl acetate (PVAC), polyvinyl alcohol (PVA), polymers of acrylic acid and their salts, polyacrylamide, polymethacrylates, vinyl pyrrolidone/vinyl acetate copolymers, polyalkylene glycols, co-block polymers of polyethylene glycol and polypropylene glycol, polyethylene oxide, hydroxylpropyl methyl cellulose (HPMC), hydroxypropyl methyl cellulose acetate succinate (HPMCAS), polyvinylcaprolactam-polyvinylacetate-polyethylene glycol graft copolymer and mixtures thereof.

8. Pharmaceutical composition according to any of the preceding claims, which is present as solid oral dosage form, preferably as granulate, capsule or tablet.

9. Pharmaceutical composition according to any of the preceding claims, which is an immediate release formulation.

10. Composition comprising trametinib or a pharmaceutically acceptable salt thereof as active ingredient and a carrier, **characterized in that** the carrier forms a matrix wherein the active ingredient is embedded and the active ingredient contains less than 1 % w/w of dimethyl sulfoxide based on the total weight of trametinib in its salt free and non-solvate form present in the composition.

11. Composition according to claim 10, wherein the carrier is selected from polymers, fats, waxes, sugars, sugar alcohols, glycol, fatty acids, salts of fatty acids, fatty acid esters and fatty alcohols.

12. Composition according to claim 10 or 11, wherein the glass transition temperature (Tg) of the intermediate is 20°C or higher.

13. Composition according to any of claims 10 to 12, wherein the weight ratio of active ingredient to carrier is in the range of 1:1 to 1:150, preferably in the range of 1:1 to 1:100.

14. Method of preparing a pharmaceutical composition according to any of claims 1 to 9 or a composition according to any of claims 10 to 13, said method comprising the steps of

(a1) dissolving the active ingredient and the carrier in a solvent, and
(b1) drying the solution obtained in step (a1), preferably by spray-drying or freeze-drying; or
(a2) mixing the active ingredient and the molten carrier, and
(b2) extruding the mixture obtained in step (a2).

## Patentansprüche

1. Pharmazeutische Zusammensetzung, umfassend Trametinib oder ein pharmazeutisch akzeptables Salz davon als Wirkstoff, mindestens einen weiteren pharmazeutischen Hilfsstoff und einen Träger, **dadurch gekennzeichnet, dass** der Träger eine Matrix bildet, in die der Wirkstoff eingebettet ist, und der Wirkstoff weniger als 1 Gew.-% Dimethylsulfoxid, basierend auf dem Gesamtgewicht von Trametinib in seiner salzfreien und Nicht-Solvat-Form, die in der Zusammensetzung vorliegt, enthält.

2. Pharmazeutische Zusammensetzung nach Anspruch 1, wobei die Zusammensetzung weniger als 1 Gew.-% Dimethylsulfoxid, basierend auf dem Gesamtgewicht von Trametinib in seiner salzfreien und Nicht-Solvat-Form, die in der Zusammensetzung vorliegt, enthält.

3. Pharmazeutische Zusammensetzung nach Anspruch 1 oder 2, wobei der Träger ausgewählt ist aus Polymeren, Fetten, Wachsen, Zuckern, Zuckeralkoholen, Glycol, Fettsäuren, Salzen von Fettsäuren, Fettsäureestern und Fettalkoholen.

4. Pharmazeutische Zusammensetzung nach einem der vorhergehenden Ansprüche, wobei der Wirkstoff in dem Träger in Form einer Feststofflösung oder Feststoffdispersion vorliegt.

5. Pharmazeutische Zusammensetzung nach einem der vorhergehenden Ansprüche, wobei der Träger eine Glasübergangstemperatur (Tg) von 20 °C oder höher hat.

6. Pharmazeutische Zusammensetzung nach einem der vorhergehenden Ansprüche, wobei der Träger hydrophil oder amphiphil ist.

7. Pharmazeutische Zusammensetzung nach einem der vorhergehenden Ansprüche, wobei der Träger ausgewählt ist aus der Gruppe, bestehend aus Polyvinylpyrrolidon, Polyvinylacetat (PVAC), Polyvinylalkohol (PVA), Polymeren von Acrylsäure und ihren Salzen, Polyacrylamid. Polymethacrylaten, Vinylpyrrolidon/Vinylacetat-Copolymeren, Polyalkylenglycolen, Co-Blockpolymeren von Polyethylenglycol und Polypropylenglycol, Polyethylenoxid, Hydroxylpropylmethylcellulose (HPMC), Hydroxypropylmethylcelluloseacetatsuccinat (HPMCAS), Polyvinylcaprolactam-Polyvinylacetat-Polyethylenglycol-Pfropfcopolymer und Gemischen davon.

8. Pharmazeutische Zusammensetzung nach einem der vorhergehenden Ansprüche, die als eine feste, orale Dosierform, bevorzugt als Granulat, Kapsel oder Tablette vorliegt.

9. Pharmazeutische Zusammensetzung nach einem der vorhergehenden Ansprüche, die eine Formulierung mit sofortiger Freisetzung ist.

10. Zusammensetzung, umfassend Trametinib oder ein pharmazeutisch akzeptables Salz davon als Wirkstoff und einen Träger, **dadurch gekennzeichnet, dass** der Träger eine Matrix bildet, in die der Wirkstoff eingebettet ist, und der Wirkstoff weniger als 1 Gew.-% Dimethylsulfoxid, basierend auf dem Gesamtgewicht von Trametinib in seiner salzfreien und Nicht-Solvat-Form, die in der Zusammensetzung vorliegt, enthält.

11. Zusammensetzung nach Anspruch 10, wobei der Träger ausgewählt ist aus Polymeren, Fetten, Wachsen, Zuckern, Zuckeralkoholen, Glycol, Fettsäuren, Salzen von Fettsäuren, Fettsäureestern und Fettalkoholen.

12. Zusammensetzung nach Anspruch 10 oder 11, wobei die Glasübergangstemperatur (Tg) des Zwischenproduktes 20 °C oder mehr beträgt.

13. Zusammensetzung nach einem der Ansprüche 10 bis 12, wobei das Gewichtsverhältnis des Wirkstoffes zu dem Träger im Bereich von 1 : 1 bis 1 : 150, bevorzugt im Bereich von 1 : 1 bis 1 : 100 liegt.

14. Verfahren zur Herstellung einer pharmazeutischen Zusammensetzung nach einem der Ansprüche 1 bis 9 oder einer Zusammensetzung nach einem der Ansprüche 10 bis 13, wobei das Verfahren die Schritte umfasst

(a1) Auflösen des Wirkstoffes und des Trägers in einem Lösungsmittel und
(b1) Trocknen der in Schritt (a1) erhaltenen Lösung, bevorzugt durch Sprühtrocknung oder Gefriertrocknung; oder
(a2) Mischen des Wirkstoffes und des geschmolzenen Trägers und
(b2) Extrudieren des in Schritte (a2) erhaltenen Gemisches.

## Revendications

1. Composition pharmaceutique comprenant du trametinib ou un sel de celui-ci acceptable sur le plan pharmaceutique en tant que principe actif, au moins un autre excipient pharmaceutique et un véhicule, **caractérisée en ce que** le véhicule forme une matrice dans laquelle le principe actif est intégré et le principe actif contient moins de 1 % en poids de sulfoxyde de diméthyle sur la base du poids total de trametinib sous sa forme dépourvue de sel et non-solvate présente dans la composition.

2. Composition pharmaceutique selon la revendication 1, où la composition contient moins de 1 % en poids de sulfoxyde de diméthyle sur la base du poids total de trametinib sous sa forme dépourvue de sel et non-solvate présente dans la composition.

3. Composition pharmaceutique selon la revendication 1 ou 2, dans laquelle le véhicule est choisi parmi des polymères, des graisses, des cires, des sucres, des alcools de sucre, du glycol, des acides gras, des sels d'acides gras, des esters d'acide gras et des alcools gras.

4. Composition pharmaceutique selon l'une quelconque des revendications précédentes, dans laquelle le principe actif est présent dans le véhicule sous la forme d'une solution solide ou d'une dispersion solide.

5. Composition pharmaceutique selon l'une quelconque des revendications précédentes, dans laquelle le véhicule a une température de transition vitreuse (Tg) de 20 °C ou plus élevée.

6. Composition pharmaceutique selon l'une quelconque des revendications précédentes, dans laquelle le véhicule est hydrophile ou amphiphile.

7. Composition pharmaceutique selon l'une quelconque des revendications précédentes, dans laquelle le véhicule est choisi dans le groupe constitué de polyvinylpyrrolidone, acétate de polyvinyle (PVAC), alcool polyvinylique (PVA), polymères d'acide acrylique et leurs sels, polyacrylamide, polyméthacrylates, copolymères de vinylpyrrolidone/acétate de vinyle, polyalkylène glycols, co-polymères séquencés de polyéthylène glycol et de polypropylène glycol,

oxyde de polyéthylène, hydroxylpropylméthylcellulose (HPMC), acétate succinate d'hydroxypropylméthylcellulose (HPMCAS), copolymère greffé de polyvinylcaprolactame-acétate de polyvinyle-polyéthylène glycol et des mélanges de ceux-ci.

**8.** Composition pharmaceutique selon l'une quelconque des revendications précédentes, qui est présente en tant que forme pharmaceutique orale solide, de préférence en que granulé, gélule ou comprimé.

**9.** Composition pharmaceutique selon l'une quelconque des revendications précédentes, qui est une formulation à libération immédiate.

**10.** Composition comprenant du trametinib ou un sel de celui-ci acceptable sur le plan pharmaceutique en tant que principe actif et un véhicule, **caractérisée en ce que** le véhicule forme une matrice dans laquelle le principe actif est intégré et le principe actif contient moins de 1 % en poids de sulfoxyde de diméthyle sur la base du poids total de trametinib sous sa forme dépourvue de sel et non-solvate présente dans la composition.

**11.** Composition selon la revendication 10, dans laquelle le véhicule est choisi parmi des polymères, des graisses, des cires, des sucres, des alcools de sucre, du glycol, des acides gras, des sels d'acides gras, des esters d'acide gras et des alcools gras.

**12.** Composition selon la revendication 10 ou 11, dans laquelle la température de transition vitreuse (Tg) de l'intermédiaire est de 20 °C ou plus élevée.

**13.** Composition selon l'une quelconque des revendications 10 à 12, dans laquelle le rapport pondéral du principe actif au véhicule est dans la plage de 1:1 à 1:150, de préférence dans la plage de 1:1 à 1:100.

**14.** Procédé de préparation d'une composition pharmaceutique selon l'une quelconque des revendications 1 à 9 ou d'une composition selon l'une quelconque des revendications 10 à 13, ledit procédé comprenant les étapes consistant à

(a1) dissoudre le principe actif et le véhicule dans un solvant, et
(b1) sécher la solution obtenue à l'étape (a1), de préférence par séchage par atomisation ou lyophilisation ; ou
(a2) mélanger le principe actif et le véhicule fondu, et
(b2) extruder le mélange obtenu à l'étape (a2).

Figure 1

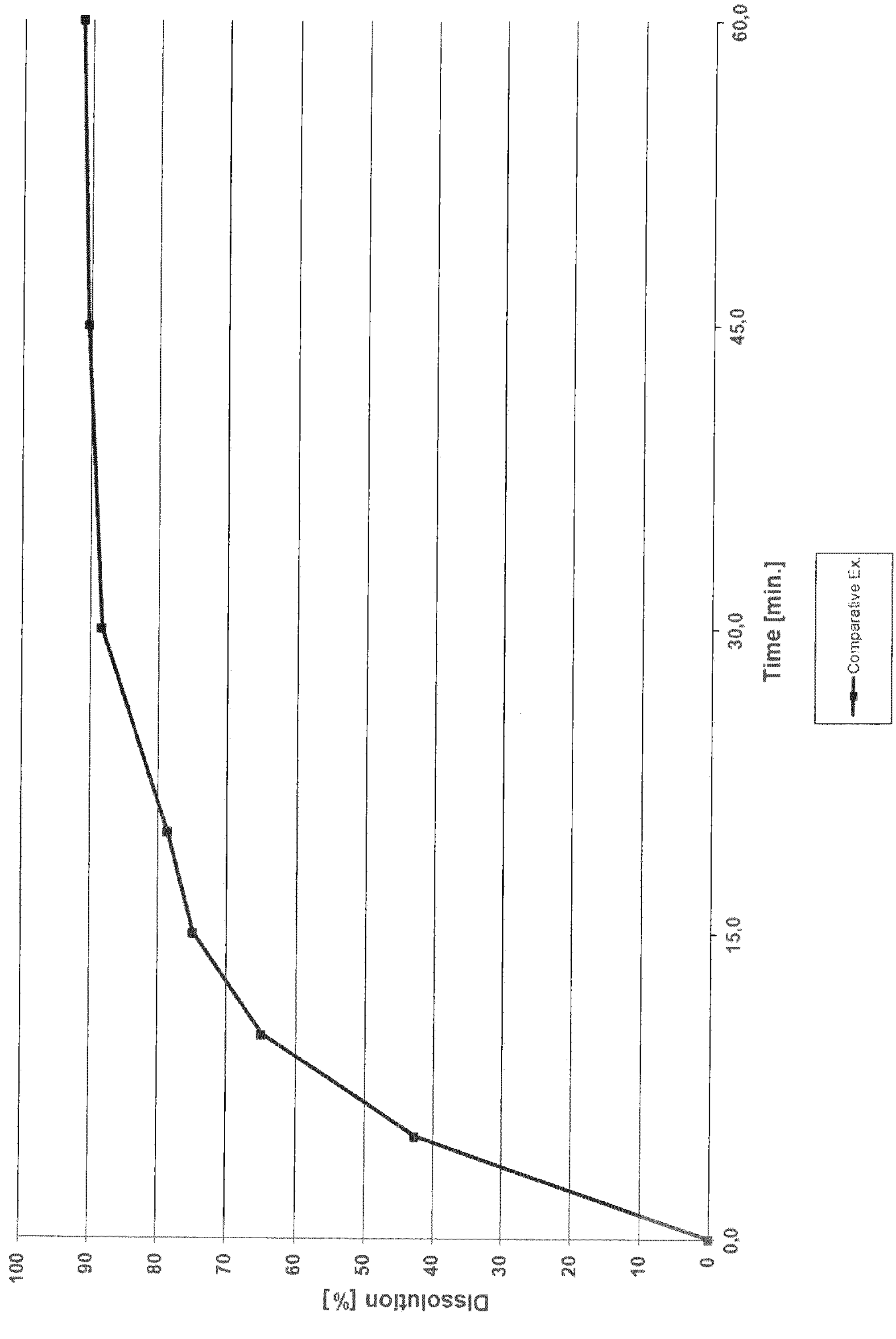
Dissolution [%]
100
90
80
70
60
50
40
30
20
10
0
0,0
15,0
30,0
45,0
60,0
Time [min.]
Comparative Ex.

Figure 2

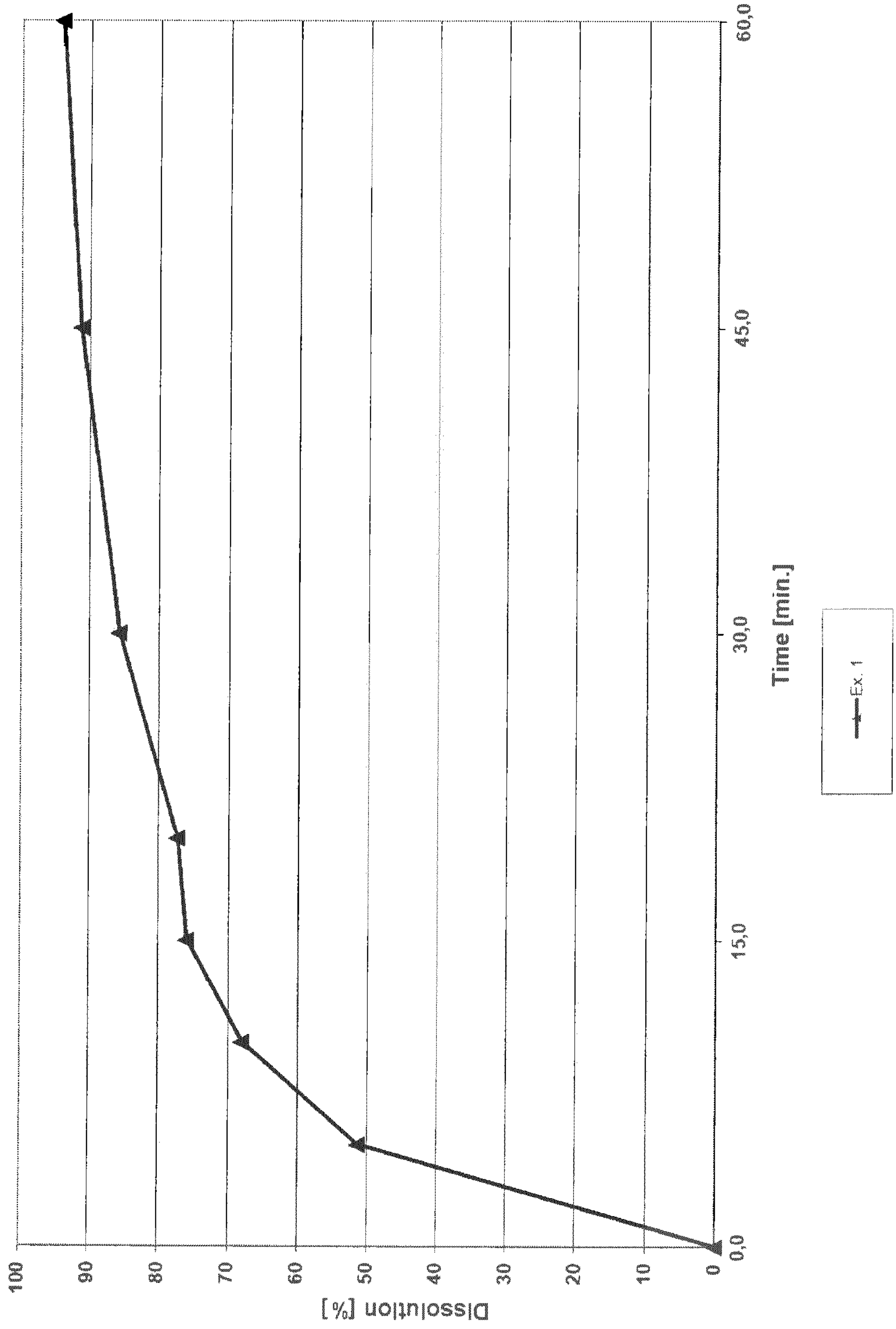
Dissolution [%]
0
10
20
30
40
50
60
70
80
90
100
Time [min.]
0,0
15,0
30,0
45,0
60,0
Ex. 1

Figure 3

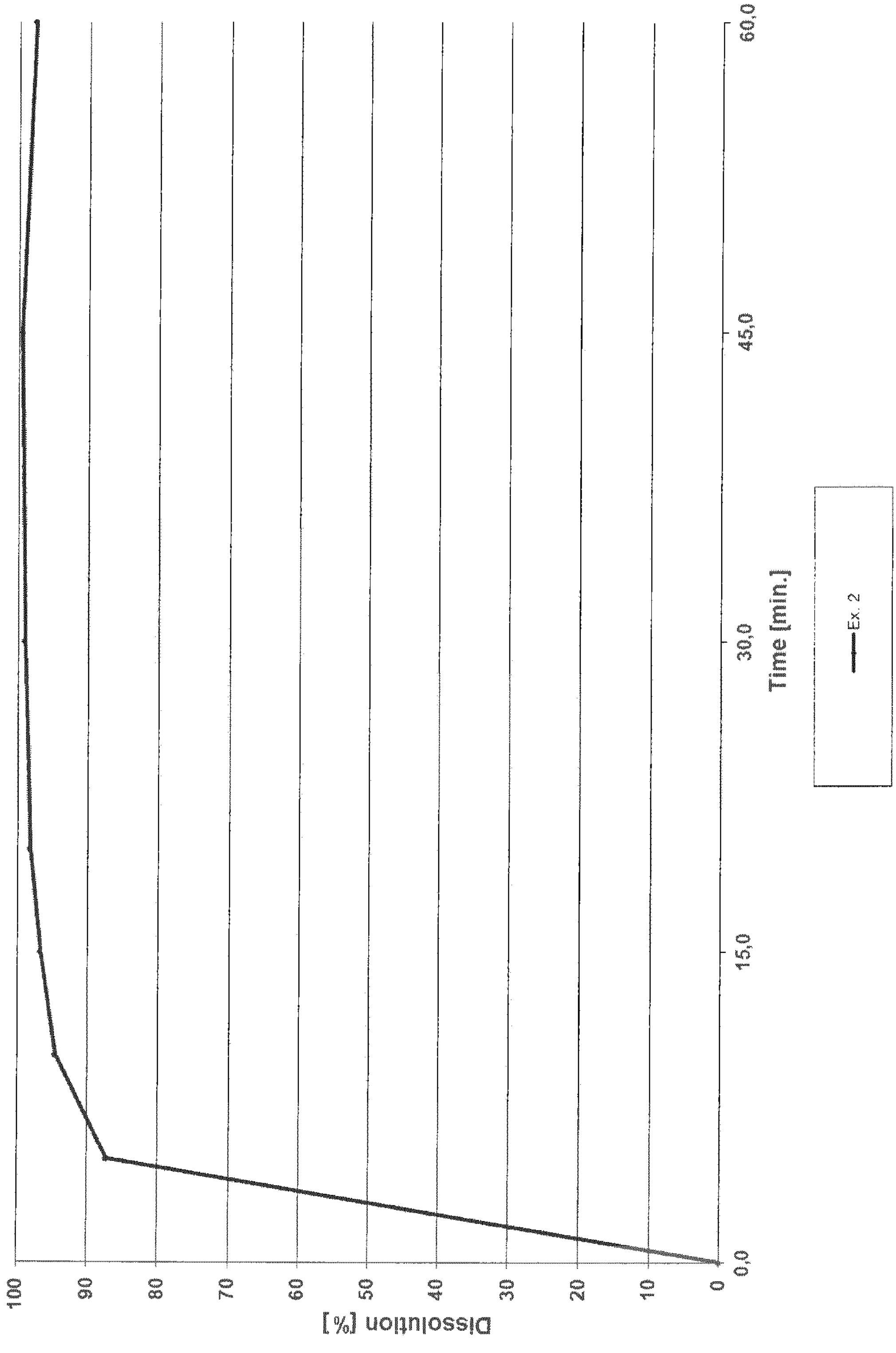
Dissolution [%]
100
90
80
70
60
50
40
30
20
10
0
0,0
15,0
30,0
45,0
60,0
Time [min.]
Ex. 2

Figure 4

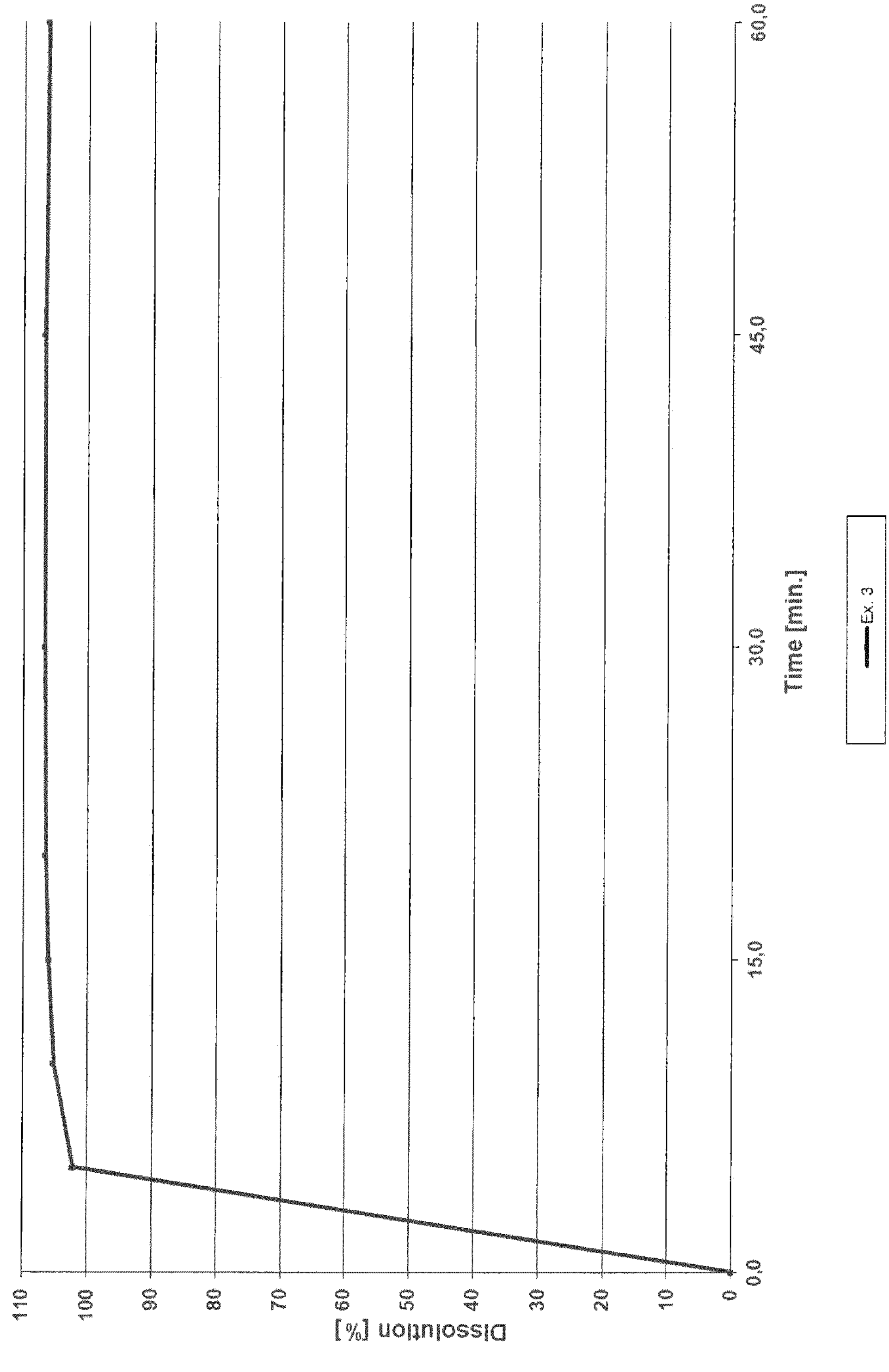
Dissolution [%]
0
10
20
30
40
50
60
70
80
90
100
110
0,0
15,0
30,0
45,0
60,0
Time [min.]
Ex. 3

Figure 5

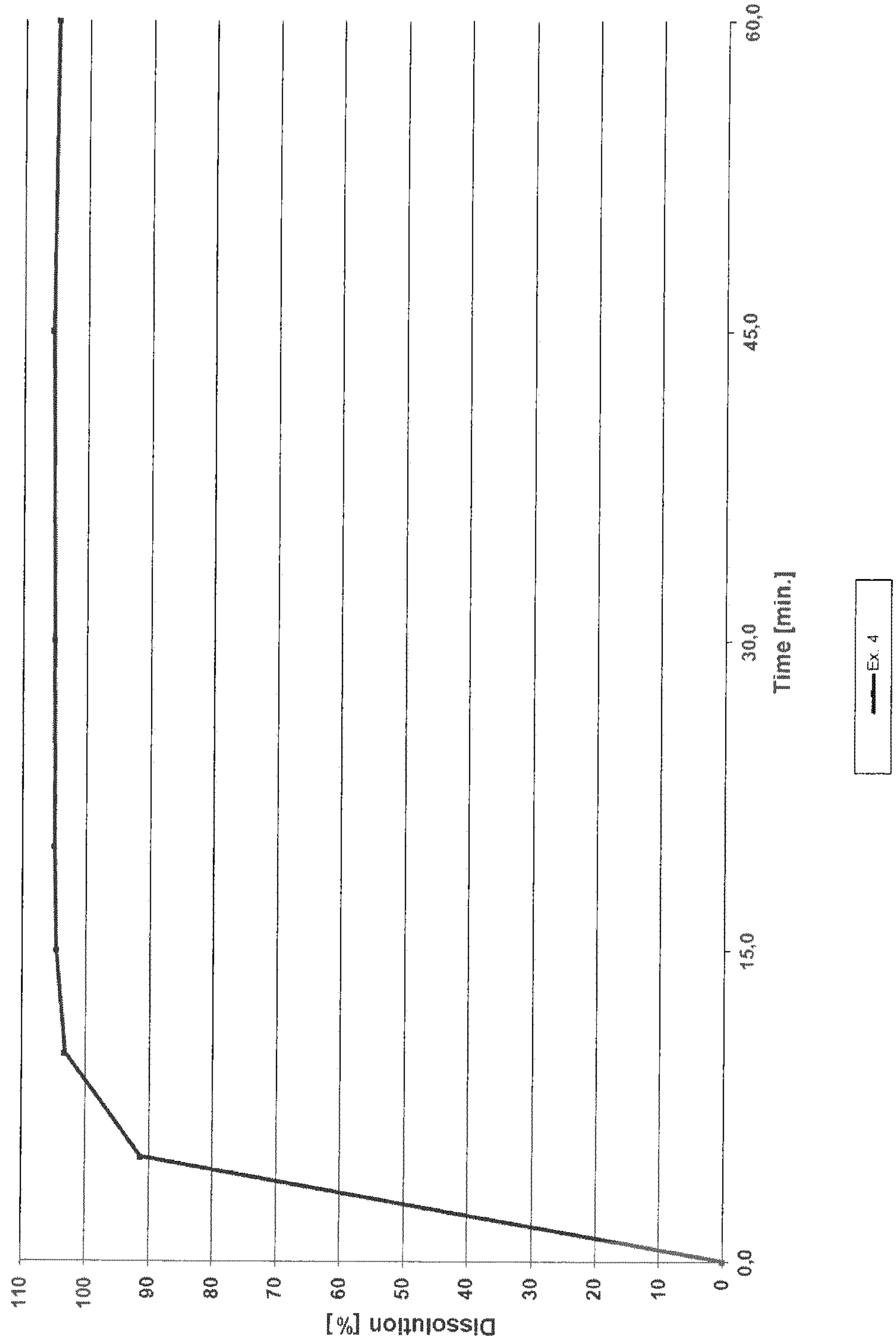
Dissolution [%]
0
10
20
30
40
50
60
70
80
90
100
110
0,0
15,0
30,0
45,0
60,0
Time [min.]
Ex. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20060014768 A
- WO 2012088033 A
- US 20120322817 A
- US 20120283278 A
- US 20130109705 A


### Non-patent literature cited in the description


- Handbook of Pharmaceutical Excipients. Pharmaceutical Press and American Pharmacists Association, 2009
- European Pharmacopoeia (Ph. Eur.)